Europäisches Patentamt

European Patent Office

Office européen des brevets

(11) Veröffentlichungsnummer: **0 257 417 B1**

## EUROPÄISCHE PATENTSCHRIFT

(45) Veröffentlichungstag der Patentschrift: **13.05.92**

(51) Int. Cl.5: **C12N 15/65**, C12N 15/76

(21) Anmeldenummer: **87111509.3**

(22) Anmeldetag: **08.08.87**

(54) **Farbmarker in Streptomyceten-Plasmiden.**

(30) Priorität: **13.08.86 DE 3627405**

(43) Veröffentlichungstag der Anmeldung:
**02.03.88 Patentblatt 88/09**

(45) Bekanntmachung des Hinweises auf die
Patenterteilung:
**13.05.92 Patentblatt 92/20**

(84) Benannte Vertragsstaaten:
**AT BE CH DE ES FR GB GR IT LI LU NL SE**

(56) Entgegenhaltungen:
**EP-A- 0 154 430**

**CHEMICAL ABSTRACTS, Band 86, 1977, Seiten 258-259, Zusammenfassung Nr. 185565e, Columbus, Ohio, US; G. HABERMEHL et al.: "Amylocyanin, the blue pigment of Streptomyces coelicolor", & NATURWISSENSCHAFTEN 1977, 64(2), 97-8**

**CHEMICAL ABSTRACTS, Band 88, 1978, Seite 256, Zusammenfassung Nr. 47257w, Columbus, Ohio, US; G. HABERMEHL et al.: "Isolation, separation and structure of the blue bacterial pigment "amylocyanin" from Streptomyces coelicolor", & Z. NATURFORSCH. B: ANORG. CHEM., ORG. CHEM.**

**1977, 32B(10), 1195-203**

**CURR. TOP. MICROBIOL. IMMUNOL., Band 96, 1982, Seiten 69-95; K.F. CHATER et al.: "Gene cloning in Streptomyces"**

**Naturwissenschaften, 1977, 64(2), 97-98**

(73) Patentinhaber: **HOECHST AKTIENGESELL-SCHAFT**
**Postfach 80 03 20**
**W-6230 Frankfurt am Main 80(DE)**

(72) Erfinder: **Muth, Günter**
**Talbrückenstrasse 91**
**W-4800 Bielefeld(DE)**
Erfinder: **Wohlleben, Wolfgang, Dr.**
**Menzelstrasse 1**
**W-4800 Bielefeld(DE)**
Erfinder: **Pühler, Alfred, Prof. Dr.**
**Am Waldschlösschen 2**
**W-4800 Bielefeld(DE)**
Erfinder: **Wöhner, Gerhard, Dr.**
**Flörsheimer Strasse 27**
**W-6093 Flörsheim am Main(DE)**
Erfinder: **Marquardt, Rüdiger, Dr.**
**Günthersburgallee 69**
**W-6000 Frankfurt am Main(DE)**

## Beschreibung

Als Farbmarker für die Klonierung in Streptomyceten ist bisher das mel-Gen bekannt (E. Katz et al., J. Gen. Microbiol. 129 (1983) 2703), das für Tyrosinase codiert und damit - über Zwischenstufen - für die Produktion des Farbstoffes Melanin verantwortlich ist. Dieses Gen ist beispielsweise in dem handelsüblichen Plasmid pIJ702 enthalten, das von der John Innes Foundation, Norwich, England, erhältlich ist und beispielsweise in D. A. Hopwood et al., Genetic Manipulation of Streptomyces - A Laboratory Manual, The John Innes Foundation, 1985, S. 292 f., beschrieben ist. Dieser Marker hat jedoch den Nachteil, daß der gebildete Farbstoff leicht in das umgebende Medium diffundiert.

Es wurde nun ein Farbstoffmarker gefunden, der in Streptomyceten exprimiert wird und im Medium eine tiefblaue bis schwarze, intensive Färbung bewirkt, die jedoch in festen Medien nur eine geringe Diffusion zeigt. Hierdurch können beim Einsatz als Inaktivierungsmarker auch sehr seltene Ereignisse leicht erkannt werden.

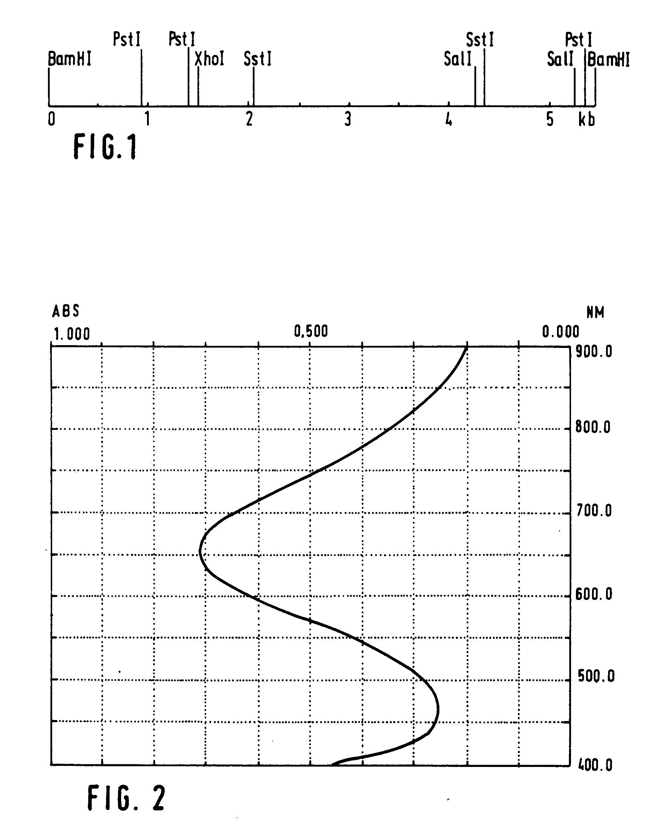

Die Erfindung betrifft somit ein Gen, das für einen blauen Farbstoff in Streptomyceten codiert und das erhältlich ist aus der Gesamt-DNA von Streptomyces coelicolor DSM 3030 durch Scheiden mit BamHI, Isolieren eines etwa 5,5 kb großen Fragments und Selektion auf Farbstoffproduktion. Dieses Gen wird durch die Restriktionskarte gemäß Figur 1 näher charakterisiert. Der Ausgangsstamm S. coelicolor DMS 3030 ist in der Europäischen Patentanmeldung mit der Veröffentlichungsnummber 0 181 562 als Produzent eines bakterienlysierenden Enzyms genannt.

Die Erfindung bezieht sich weiterhin auf die Verwendung des erfindungsgemäßen Gens als Marker, insbesondere als Inaktivierungs-Marker, in Streptomyceten-Plasmiden.

Weitere Aspekte der Erfindung und ihre bevorzugten Ausgestaltungen ergeben sich aus der folgenden Beschreibung bzw. aus den Patentansprüchen.

Zur Isolierung des Markergens isoliert man die Gesamt-DNA aus dem Stamm Streptomyces coelicolor DMS 3030 durch Scheiden mit dem Restriktionsenzym BamHI und "shot gun"-Klonierung in einen geeigneten Vektor, Transformation eines Strptomyceten-Empfängerstamms und Selektion auf Farbstoffproduktion. Die positive Klone enthalten ein etwa 5,5 kb DNA-Fragment aus DSM 3030.

Der gebildete blaue Farbstoff ist - im Gegensatz zu dem im Wildtyp gebildeten wasserlöslichen Farbstoff - nicht wasserlöslich, aber gut wasserdispergierbar. Die Figur 2 zeigt das UV-Absorptionsspektrum eines wäßrigen Kulturüberstands. Das Absorptionsmaximum liegt bei 660 nm und das Minimum bei 460 nm. Die fast schwarze Koloniemorphologie kommt durch eine hohe Farbstoffkonzentration zustande. In einem Festmedium bleibt der überwiegende Teil des Farbstoffs am Mycel gebunden und nur ein geringer Teil diffundiert in das Medium. Unter dem Mikroskop zeigt sich ein charakteristisches Bild: Der Farbstoff liegt in dicht gedrängten kugelförmigen Körpern am Mycel und läßt somit die Kolonie fast schwarz erscheinen. In Flüssigkultur (Tryptic soya broth, "Lysemedium A", Europäische Patentanmeldung mit der Veröffentlichungsnummer 0 158 872, S. 6) erhält man nach etwa 3 Tagen einen intensiv blau gefärbten Kulturüberstand. Auch in festen Medien erfolgt die Farbstoffproduktion unabhängig vom eingesetzten Medium ("R2YE" (Hopwood et al., a.a.O.), Sporulationsmedium, (Deutsche Offenlegungsschrift 3 331 860, Beispiel 1, drittes Medium), "Penassay", "Penassay" mit Antibiotica-Zusatz). Der blaue Farbstoff wird bereits zu Beginn des Koloniewachstums gebildet und ist somit offensichtlich kein Produkt des sekundären Stoffwechsels.

Das 5,5 kb-Fragment gemäß Figur 1 zeigt eine Reihe von Schnittstellen, die für Subklonierungsexperimente geeignet sind. Es enthält keine Schnittstellen für die Enzyme BclI, BglII, HindIII, HpaI, EcoRI, EcoRV und ClaI und mehr als 4 Schnittstellen für SstII und PvuII.

Als Wirtsstämme für Klonierungsexperimente eignen sich alle bisher untersuchten Streptomyceten-Arten, wobei jedoch die Menge des produzierten Farbstoffs nicht nur von Art zu Art, sondern auch von Stamm zu Stamm schwanken kann. Aufgrund der Intensität der Färbung eignet sich das erfindungsgemäße Markersystem aber auch vorzüglich in den Stämmen, die geringere Expression zeigen.

Durch die aufgezeigten Vorteile eignet sich der erfindungsgemäße Marker nicht nur generell für Klonierungsexperimente, sondern speziell zum Aufspüren von Stoffwechselwegen in Streptomyceten, die bekanntlich wichtige Produzenten von Antibiotika und auch anderen Stoffwechselprodukten sind.

In den folgenden Beispielen wird die Erfindung näher erläutert. Prozentangaben und Teile beziehen sich hierbei auf das Gewicht, sofern keine anderen Angaben gemacht sind.

Die Figuren - mit Ausnahme der Polylinkerregionen - sind maßstabsgerecht.

Beispiel 1: Herstellung des Vektors pGM4

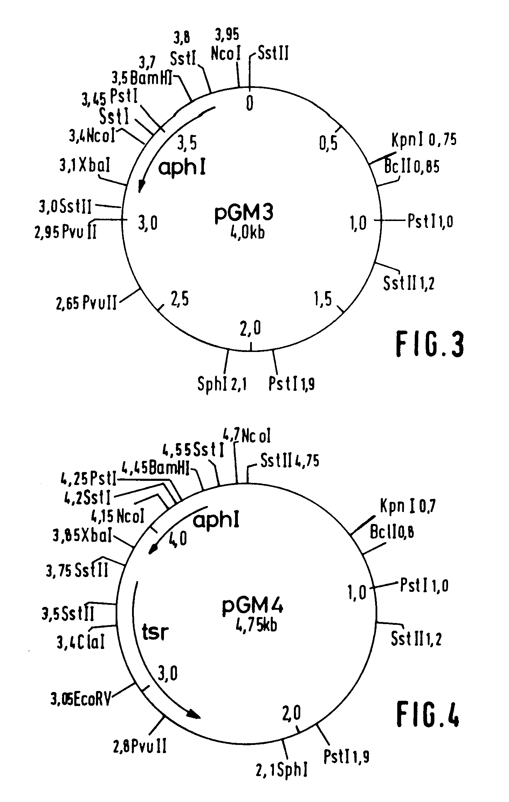

Aus dem Plasmid pGM1 (Europäische Patentanmeldung mit der Veröffentlichungsnummer 0 158 872, Figur 2) wird durch partielle Verdauung mit SstII ein 3,0 kb Fragment gewonnen. Aus dem Plasmid pSLE16 (Europäische Patentanmeldung mit der Veröffentlichungsnummer 0 158 201, Figur 18) wird durch Scheiden mit SstII das 1 kb-Fragment gewonnen, das das Neomycin-Resistenzgen aphI enthält. Durch Ligieren der beiden Fragmente erhält man das Plasmid pGM3 (Figur 3).

Aus pSLE41 (EP-A2 0 158 201, Fig. 20) wird außerdem durch Scheiden mit BclII ein 1 kb-Fragment isoliert, dessen überstehende Enden mit Klenow-Polymerase aufgefüllt werden.

pGM3 wird nun mit PvuII geschnitten und das 0,3 kb-Fragment entfernt. Das Restplasmid wird nun mit dem stumpfendig gemachten BclI-Fragment ligiert, wobei das Plasmid pGM4 erhalten wird (Figur 4).

Beispiel 2: Herstellung einer Genbank

S. coelicolor DSM 3030 wird lysiert und die DNA in bekannter Weise isoliert. Diese wird mit BamHI total verdaut. Das Plasmid pGM4 (Figur 4) wird mit BamHI beschnitten, mit Alkalischer Phosphatase behandelt und mit den BamHI-Fragmenten ligiert. Die so erhaltene Plasmid-Population wird in den Emfängerstamm S. lividans TK 23 (erhältlich bei John Innes Foundation) transformiert. Man erhält beim Einsatz von 1 µg Ligationsgemisch etwa 20000 Thiostrepton-resistente Transformanten, von denen 80 % Neomycin-sensitiv sind und folglich ein Insert enthalten.

Beispiel 3: Isolierung des Markergens

Intensiv blau-schwarz gefärbte Transformanten-Kolonien wurden abgetrennt und die Plasmid-DNA aus diesen Klonen isoliert. Eine nachfolgende Retransformation nach S. lividans TK 23 erbrachte nur blau-schwarz gefärbte Kolonien.

Eine Charakterisierung des Plasmid-DNA ergab, daß ein 5,5 kb BamHI-Fragment, das durch die Figur 1 charakterisiert ist, in das aphI-Gen von pGM4 insertiert war. Dieses Plasmid erhielt die Bezeichnung pGM98.

Beispiel 4: Transformation anderer Streptomyceten-Arten

Das Plasmid pGM98 wurde in verschiedene Stämme von S. prasinus (DSM 40099), S. viridochromogenes (DSM 40736 = DSM 4112) und S. ghanaensis (DSM 2932, ATCC 14672) transformiert.

In allen Stämmen konnte eine Farbstoffbildung beobachtet werden, die jedoch in S. prasinus etwas geringer war als in S. lividans, aber etwas intensiver war als in S. ghanaensis. Ein Plasmidverlust wurde in keinem Falle beobachtet.

Das Plasmid pGM98 wurde auch in S. coelicolor DSM 3030 transformiert. In diesem Fall setzt die Farbstoffbildung sofort ein, während sie im Ausgangsstamm (Wildtyp) erst nach drei Tagen zu beobachten ist. Außerdem erfolgt die Farbstoffproduktion auch auf Medien, auf denen der Wildtyp keinen blauen Farbstoff bildet.

## Patentansprüche

**Patentansprüche für folgende Vertragsstaaten : BE, CH, DE, FR, GB, GR, IT, LI, LU, NL, SE**

1. Für einen blauen Farbstoff in Streptomyceten codierendes Gen, erhältlich aus der Gesamt-DNA von Streptomyces coelicolor DSM 3030 durch Schneiden mit BamHI, Klonieren eines etwa 5,5 kb großen Fragments und Selektion auf Farbstoffproduktion.

2. Gen nach Anspruch 1, gekennzeichnet durch die Restriktionskarte nach Figur 1.

3. Verwendung des Gens nach Anspruch 1 oder 2 als Marker in Streptomyceten-Plasmiden.

4. Verwendung des Gens nach Anspruch 1 oder 2 als Inaktivierungs-Marker in Streptomyceten-Plasmiden.

**Patentansprüche für folgende Vertragsstaaten : AT, ES**

1. Verfahren zur Gewinnung eines für einen blauen Farbstoff in Streptomyceten codierenden Gens, dadurch gekennzeichnet, daß man die Gesamt-DNA von Streptomyces coelicolor DSM 3030 mit BamHI schneidet, ein etwa 5,5 kb großes Fragment kloniert und auf Farbstoffproduktion selektiert.

2. Verfahren nach Anspruch 1, dadurch gekennzeichnet, daß man das durch die Restriktionskarte nach Figur 1 definierte Fragment kloniert.

3. Verwendung des nach Anspruch 1 oder 2 erhältlichen Gens als Marker in Streptomyceten-Plasmiden.

4. Verwendung des nach Anspruch 1 oder 2 erhältlichen Gens als Inaktivierungs-Marker in Streptomyceten-Plasmiden.

## Claims

**Claims for the following Contracting States : BE, CH, DE, FR, GB, GR, IT, LI, LU, NL, SE**

1. A gene coding for a blue coloring agent in Streptomycetes, obtainable from the total DNA of Streptomyces coelicolor DSM 3030 by cutting with BamHI, cloning a fragment about 5.5 kb in size, and selection for production of coloring agent.

**2.** The gene as claimed in claim 1, which has the restriction map shown in Figure 1.

**3.** The use of the gene as claimed in claim 1 or 2 as marker in Streptomycetes plasmids.

**4.** The use of the gene as claimed in claim 1 or 2 as inactivation marker in Streptomycetes plasmids.

**Claims for the following Contracting States : AT, ES**

**1.** A process for obtaining a gene coding for a blue coloring agent in Streptomycetes, which comprises cutting the total DNA of Streptomyces coelicolor DSM 3030 with BamHI, cloning a fragment about 5.5 kb in size, and selection for production of coloring agent.

**2.** The process as claimed in claim 1, wherein the fragment defined by the restriction map shown in Figure 1 is cloned.

**3.** The use of the gene obtainable as claimed in claim 1 or 2 as marker in Streptomycetes plasmids.

**4.** The use of the gene obtainable as claimed in claim 1 or 2 as inactivation marker in Streptomycetes plasmids.

**Revendications**
**Revendications pour les Etats Contractants suivants : BE, CH, DE, FR, GB, GR, IT, LI, LU, NL, SE**

**1.** Gène codant pour un colorant bleu dans des Streptomyces, pouvant être obtenu à partir de l'ADN total de Streptomyces coelicolor DSM 3030 par coupure par BamHI, clonage d'un fragment d'environ 5,5 kb et sélection sur la base de la production de colorant.

**2.** Gène selon la revendication 1, caractérisé par la carte de restriction selon la figure 1.

**3.** Utilisation du gène selon la revendication 1 ou 2, en tant que marqueur dans des plasmides de Streptomyces.

**4.** Utilisation du gène selon la revendication 1 ou 2, en tant que marqueur d'inactivation dans des plasmides de Streptomyces.

**Revendications pour les Etats Contractants suivants : AT, ES**

**1.** Procédé pour l'obtention d'un gène codant pour un colorant bleu dans des Streptomyces, caractérisé en ce que l'on coupe par BamHI l'ADN total de Streptomyces coelicolor DSM 3030, on clone un fragment d'environ 5,5 kb et on sélectionne sur la base de la production de colorant.

**2.** Procédé selon la revendication 1, caractérisé en ce que l'on clone le fragment défini par la carte de restriction selon la figure 1.

**3.** Utilisation du gène pouvant être obtenu selon la revendication 1 ou 2, en tant que marqueur dans des plasmides de Streptomyces.

**4.** Utilisation du gène pouvant être obtenu selon la revendication 1 ou 2, en tant que marqueur d'inactivation dans des plasmides de Streptomyces.

FIG.1

FIG. 2

FIG.3

FIG.4